Europäisches Patentamt

European Patent Office

Office européen des brevets

(11) Veröffentlichungsnummer: **0 437 229 A1**

# (12) EUROPÄISCHE PATENTANMELDUNG

(21) Anmeldenummer: 91100157.6

(51) Int. Cl.$^5$: **A61B 1/00, A61B 1/12**

(22) Anmeldetag: 05.01.91

(30) Priorität: 09.01.90 DE 4000410

(43) Veröffentlichungstag der Anmeldung:
17.07.91 Patentblatt 91/29

(84) Benannte Vertragsstaaten:
AT BE CH DE DK ES FR GB GR IT LI LU NL SE

(71) Anmelder: Bäuerle, Dieter
Mörikestrasse 24
W-7000 Stuttgart 1 (DE)
Anmelder: Schmidt, Karlheinz
Mörikestrasse 24
W-7000 Stuttgart 1 (DE)

(72) Erfinder: Grund, Karl Ernst, Dr.
Auschbachstrasse 22
W-7400 Tübingen (DE)

(74) Vertreter: Patentanwälte Phys. Bartels
Dipl.-Ing. Fink Dr.-Ing. Held
Lange Strasse 51
W-7000 Stuttgart 1 (DE)

(54) Endoskop.

(57) Bei einem Endoskop (1) mit einem Instrumentierkanal (2) weist dieser an seinem äußeren Ende einen Abzweig (5) auf, an den die Druckseite eines Pumpenaggregates (7) angeschlossen ist.

EP 0 437 229 A1

Fig.1

# ENDOSKOP

Die Erfindung betrifft ein Endoskop mit zugänglichem Ende seines Instrumentierkanals.

Bei den bekannten Endoskopen ist es notwendig, daß der Instrumentierkanal frei ist, sich also im Instrumentierkanal kein Instrument befindet, wenn man, um eine Absaugung oder eine Insufflation vorzunehmen oder zu spülen, durch den Instrumentierkanal saugt bzw. Wasser oder Luft einführt, weil hierzu eine Handspritze an das frei zugängliche Ende des Instrumentenkanals angesetzt werden muß. Ebenso ist es unumgänglich, das augenblicklich benutzte Instrument aus dem Instrumenkanal herauszuziehen, wenn mittels einer Handspritze Flüssigkeit eingespritzt werden soll, um den Arbeitsbereich von die Sicht behindernden Partikeln freizuspülen.

Der Erfindung liegt die Aufgabe zugrunde, ein Endoskop zu schaffen, das dem Benutzer bessere Arbeitsbedingungen zu bieten vermag. Diese Aufgabe löst ein Endoskop mit den Merkmalen des Anspruches 1.

Dank des Abzweiges am zugänglichen Endes des Instrumentierkanals, an den die Druckseite eines Pumpenaggregates angeschlossen ist, ist es möglich, während der Arbeit mit dem sich im Instrumentierkanal befindenden Instrument durch den Instrumentierkanal hindurch das im Bedarfsfalle vom Pumpenaggregat geförderte Substrat einzuführen.

Dabei kann es sich um Luft oder Flüssigkeit, z.B. NaCl-Lösung oder Wasser handeln. Besonders vorteilhaft ist in vielen Fällen jedoch ein Luft-Flüssigkeit-Gemisch. Bei der beschriebenen Ausführungsform weist deshalb das Pumpenaggregat sowohl eine Wasserpumpe als auch eine Luftpumpe auf, die gleichzeitig oder alternierend, automatisch oder schaltgesteuert aktiviert werden können, so daß durch den Instrumentierkanal hindurch ein Luft-Flüssigkeits-Gemisch zugeführt wird. Aber auch dann, wenn nur Flüssigkeit oder Luft zugeführt werden kann, ergeben sich erheblich verbesserte Arbeitsbedingungen, weil die Einleitung synchron zu der Arbeit mit dem eingeführten Instrument erfolgen kann.

Besonders vorteilhaft ist es, sowohl die Fluidzufuhr durch den Instrumentierkanal hindurch als auch die Absaugung beliebig steuerbar ausführen zu können, indem beispielsweise die Steuerung des Pumpenaggregates mittels eines Fußschalters vorgenommen wird und die Absaugung von Hand gesteuert wird. Es können dann problemlos während der gesamten Arbeit gute Sichtverhältnisse aufrechterhalten werden, weil entsprechend dem Bedarf Luft zur notwendigen Entfaltung des Op-Gebietes und Flüssigkeit für einen Spülvorgang zugeführt werden können, mittels dessen Blut oder anderes störendes Material beseitigt werden kann.

Bei einer vorteilhaften Ausführungsform ist jede vorhandene Pumpe über ein steuerbares Ventil mit dem Abzweig verbunden. Vorteilhafterweise liegt dabei jedes Ventil in einer von der Pumpe zu einem Verbindungsstück führenden Leitung, von dem aus eine einzige, gemeinsame Sammelleitung zum Abzweig führt, und zwar auch dann, wenn an das Verbindungsstück sowohl eine Wasserpumpe als auch eine Luftpumpe angeschlossen sind.

Zur Ansteuerung der Ventile, bei denen es sich zweckmäßigerweise um Magnetventile handelt, ist vorteilhafterweise ein Fußschalter vorgesehen. Der Benutzer des Endoskopes hat dann die Hände frei für die Handhabung des Endoskops und des in dieses eingeführten Instrumentes.

Soll die Förderleistung der Pumpe oder Pumpen verändert werden, dann kann die entsprechende Steuerung ebenfalls mit dem Fußschalter erfolgen, sofern man nicht, was in der Regel ausreichend sein wird, eine manuelle Einstellbarkeit der Förderleistung vorsieht.

Bei einer bevorzugten Ausführungsform ist die Wasserpumpe an einen lösbar mit dem Pumpenaggregat verbundenen Flüssigkeitsvorratsbehälter angeschlossen, der mit einem Sensor- oder Schwimmerschalter ausgerüstet ist, um ein Alarmsignal zu erzeugen und/oder die Pumpe abzuschalten, wenn sich nur noch eine Mindestmenge an Flüssigkeit in dem Vorratsbehälter befindet.

Im folgenden ist die Erfindung anhand eines in der Zeichnung dargestellten Ausführungsbeispiels im einzelnen erläutert.

Es zeigen

Fig. 1 eine persprektivisch und unvollständig dargestellte Ansicht des Ausführungsbeispiels,

Fig. 2 ein Schaltbild des zugehörigen Pumpenaggregates,

Fig. 3 einen Schnitt durch den zugehörigen Wasservorratsbehälter.

Ein in bekannter Weise ausgebildetes, starres oder flexibles Endoskop 1, weist einen Instrumentierkanal 2 auf, der einerseits am vorderen Ende des Endoskops und andererseits nahe dessen hinterem Ende in einem Stutzen 3 endet, der vom Schaft des Endoskopes abzweigt. Bei den bekannten Endoskopen bildet das offene Ende dieses Stutzens 3 das zugängliche Ende des Instrumentierkanals, durch das hindurch ein Instrument 4 eingeführt werden kann.

An diesen Stutzen 3 ist eine Verlängerung mit einem Abzweig 5 angesetzt. Im Ausführungsbeispiel handelt es sich um ein T-Stück, dessen Querteil den Instrumentierkanal geradlinig mit mindestens gleichem Durchmesser verlängert. Das zugängliche Ende des Instrumentierkanals 2 wird also nicht durch den Stutzen 4, sondern durch den Querteil des T-Stückes gebildet, welcher den Stutzen 3 verlängert. In den Kanal des Querteils mündet der Kanal des den Abzweig 5 bildenden Längsteils, an den eine flexible Schlauchleitung 6 angeschlossen ist.

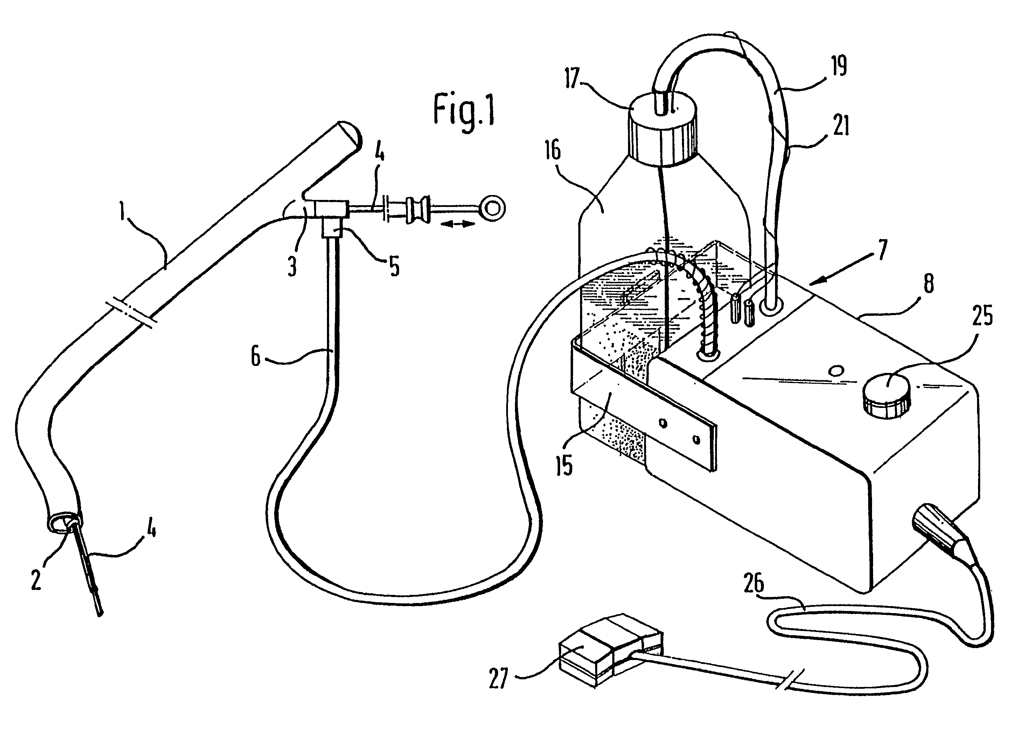

Wie Fig. 1 zeigt, führt diese flexible Schlauchleitung 6 zu einem als Ganzes mit 7 bezeichneten Pumpenaggregat. Dieses Pumpenaggregat weist ein im Ausführungsbeispiel quaderförmiges Gehäuse 8 auf, in dem eine von einem Elektromotor 9 antreibbare Wasserpumpe 10 sowie eine von einem anderen Elektromotor 11 antreibbare Luftpumpe 12 enthalten ist. Ferner enthält im Ausführungsbeispiel das Gehäuse 8 in der Ansaugleitung der Wasserpumpe ein Filter 14 und in der Ansaugleitung der Luftpumpe 12 ein Filter 13. Über die eine Stirnseite des Gehäuses 8 steht ein Bügel 15 über, an dessen Jochteil sich mittig der eine Schenkel eines Trägerwinkels anschließt, dessen anderer Schenkel mit dem Boden des Gehäuses 8 verbunden ist und eine Stellfläche für eine im Querschnitt rechteckförmige, den Flüssigkeitsvorrat enthaltende Flasche 16 bildet. Diese Flasche 16 wird von dem Bügel 15 umfaßt. Sie ist daher leicht auswechselbar mit dem Gehäuse 8 zu einer Einheit verbunden. Die Halterung ist so ausgelegt, daß als Flasche die üblichen, genormten Kunststoffflaschen für medizinische Infusionslösungen verwendet werden können. Damit und bei Verwendung einer Rollerpumpe sind die hygienischen Anforderungen optimal erfüllt.

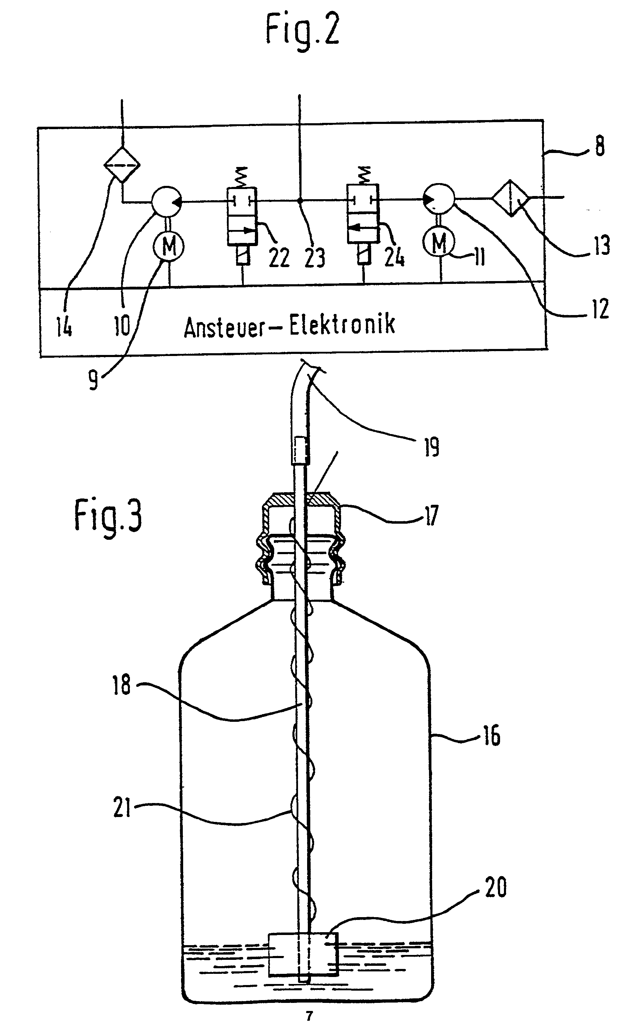

Durch einen die Flasche 16 im Bereich ihres Halses verschließenden, abnehmbaren Verschluß 17 hindurch ist in die Flasche 16 ein Rohr 18 eingeführt, das nur wenig über dem Boden der Flasche 16 endet. Mit dem aus dem Verschluß 17 herausragenden Ende des Rohres 18 ist ein Schlauch 19 verbunden, der zum Ansaugstutzen des Filters 14 führt. Auf dem Rohr 18 ist im Inneren der Flasche in Rohrlängsrichtung verschiebbar ein Sensor oder ein Schwimmerschalter 20 gelagert, der über eine flexible Verbindungsleitung 21 mit einer im Gehäuse 8 enthaltenen Elektronik verbunden ist. Der Sensor oder Schwimmerschalter 20 löst ein Signal aus, wenn er die in Fig. 3 dargestellte untere Endlage einnimmt, in welcher nur noch ein geringer Flüssigkeitsvorrat sich in der Flasche 16 befindet.

Das Einsatzteil der Flasche 16 einschließlich Anschlußschläuche und Filter ist als austauschbares oder sterilisierbares Teil ausgeführt.

Wie Fig. 2 zeigt, ist die Druckseite der Wasserpumpe 10 über ein Magnetventil 22 an einen T-Verbinder 23 angeschlossen, mit dem andererseits über ein zweites Magnetventil 24 die Druckseite der Luftpumpe 12 verbunden ist. Mit dem dritten Anschluß des T-Verbinders 23 ist als gemeinsame Sammelleitung die zum Abzweig 5 führende flexible Schlauchleitung 6 verbunden. Im Ausführungsbeispiel sind die Magnetventile 22 und 24 im Gehäuse 8 angeordnet. Sie können aber auch beispielsweise im Anschlußstück 5 untergebracht sein.

Ein nicht dargestelltes Steckernetzteil liefert die für den Betrieb der beiden Elektromotoren 9 und 11, der Magnetventile 22 und 24 sowie der Elektronik die elektrische Energie.

Im Ausführungsbeispiel ist für beide Elektromotoren eine elektronische Drehzahlsteuerschaltung vorgesehen, mittels deren, im Ausführungsbeispiel mit Hilfe eines manuell betätigbaren Drehknopfes 25, der auf der Oberseite des Gehäuses 8 vorgesehen ist, die Motordrehzahl zwischen einem Minimalwert und einem Maximalwert frei wählbar eingestellt werden kann. Zur alternierenden oder gleichzeitigen Applikation von Flüssigkeit und Luft enthält das Gehäuse 8 eine Automatikschaltung. Astabile Multivibratoren mit in weiten Grenzen einstellbarem Impuls/Pausenverhältnis steuern mit einstellbarem Verhältnis der Zeitintervalle und Fördermengen die Förderung von Luft und Flüssigkeit über nachgeschaltete Treiberstufen. Eine ebenfalls im Gehäuse 8 vorhandene Sicherheitsschaltung ermöglicht es, die durch ein einmaliges Einschalten applizierte Luft-und/oder Flüssigkeitsmenge auf einen wählbaren Wert zu begrenzen. Beispielsweise kann eine Förderzeit von 10 sec. für die Flüssigkeit und 3 sec. für die Luft gewählt werden.

Über ein flexibles Kabel 26 ist mit dem Gehäuse 8 ein Fußschalter 27 verbunden, mittels dessen die beiden Elektromotoren 9 und 11 und die Magnetventile 22 und 24 entweder einzeln oder gemeinsam eingeschaltet werden können. Es ist auch möglich, den Fußschalter 27 so auszubilden, daß mit ihm die Förderleistung der Wasserpumpe 10 und/oder der Luftpumpe 12 gesteuert werden kann, beispielsweise in der Weise, daß in der Einschaltstellung mit zunehmender Betätigungskraft die Förderleistung zunimmt. Die im Ausführungsbeispiel in das Gehäuse 8 integrierte elektrische Automatikschaltung, mit der eine zeitgesteuerte alternierende oder durchflußgesteuerte synchrone Einschaltdauer beider Pumpen und Ventile realisiert werden kann, kann ebenfalls durch den Fußschalter 27 oder mittels eines Schalters direkt am Gehäuse 8 aktiviert werden. Dank des Fußschalters 27 hat der Benutzer des Endoskopes 1 beide Hände für das Endoskop und das in dieses eingeführte Instrument 4 frei. Da außerdem je nach Bedarf während des Gebrauchs des Endoskopes und des Instrumentes 4 Flüssigkeit und/oder Luft durch den Instrumentierkanal 2 hindurch eingeführt werden kann, sind die Arbeitsbedingungen optimal.

Alle in der vorstehenden Beschreibung erwähnten sowie auch die nur allein aus der Zeichnung entnehmbaren Merkmale sind als weitere Ausgestaltungen Bestandteile der Erfindung, auch wenn sie nicht besonders hervorgehobenund insbesondere nicht in den Ansprüchen erwähnt sind.

**Patentansprüche**

1. Endoskop mit einem Instrumentierkanal, dadurch gekennzeichnet, daß der Instrumentierkanal (2) an seinem äußeren Ende einen Abzweig (5) aufweist, an den die Druckseite eines Pumpenaggregates (7) angeschlossen ist.

2. Endoskop nach Anspruch 1, dadurch gekennzeichnet, daß vom Abzweig (5) wenigstens ein flexibler Schlauch (6) zum Pumpenaggregat (7) führt.

3. Endoskop nach Anspruch 1 oder 2, dadurch gekennzeichnet, daß das Pumpenaggregat (5) eine Wasserpumpe (9, 10) und eine Luftpumpe (11, 12) enthält.

4. Endoskop nach Anspruch 3, dadurch gekennzeichnet, daß sowohl die Wasserpumpe (9, 10) als auch die Luftpumpe (11, 12) über je ein steuerbares Ventil (22, 24) mit dem Abzweig (5) verbunden ist.

5. Endoskop nach Anspruch 4, dadurch gekennzeichnet, daß die beiden Ventile (22, 24) je in einer von der zugeordneten Pumpe (10, 11) zu einem Verbindungsstück (23) führenden Leitung liegen, von dem aus eine gemeinsame Sammelleitung (6) zum Abzweig (5) führt, oder in den Abzweig (5) integriert sind.

6. Endoskop nach einem der Ansprüche 1 bis 5, gekennzeichnet durch einen Kombinationsfußschalter (27) mit drei Funktionen zur Steuerung des Antriebs (9, 11) der Pumpen (10, 12) und-/oder der Ventile (22, 24).

7. Endoskop nach einem der Ansprüche 1 bis 6, dadurch gekennzeichnet, daß das Pumpenaggregat (7) eine Steuer- oder Regelschaltung für eine Veränderung der Förderleistung der Pumpe (10) oder Pumpen (10, 12) aufweist.

8. Endoskop nach einem der Ansprüche 1 bis 7, gekennzeichnet durch eine Automatikschaltung zur alternierenden oder gleichzeitigen Applikation von Flüssigkeit und Luft mit einstellbarem Verhältnis der Zeitintervalle und Fördermengen.

9. Endoskop nach einem der Ansprüche 1 bis 8, gekennzeichnet durch eine Sicherheitsschaltung, welche die durch einmaliges Einschalten applizierte Flüssigkeits- und/oder Luftmenge begrenzt.

10. Endoskop nach einem der Ansprüche 3 bis 9, dadurch gekennzeichnet, daß die Wasserpumpe (10) an einen Flüssigkeitsbehälter (16), vorzugsweise eine genormte Kunststoffflasche, angeschlossen ist, der vorzugsweise mittels eines Sensors oder Schwimmerschalters (20) ausgerüstet ist.

11. Endoskop nach Anspruch 10, dadurch gekennzeichnet, daß der Flüssigkeitsbehälter (16) mit dem das Pumpenaggregat (7) enthaltenden Gehäuse (8) zu einer lösbaren Baueinheit vereinigt ist.

Fig.1

# Fig.2

# Fig.3

Ansteuer—Elektronik

| | Europäisches Patentamt | EUROPÄISCHER RECHERCHENBERICHT | | Nummer der Anmeldung EP 91 10 0157 |

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | DE-A-3 616 193 (OLYMPUS OPTICAL CO., LTD) * Seite 5, Zeilen 11-24; Seite 7, Zeile 10 - Seite 8, Zeile 7; Abbildungen 3-7 * --- | 1 | A 61 B 1/00 A 61 B 1/12 |
| Y | DE-A-2 912 303 (MEDO KENKYUSHO K.K.) * Seite 15, Zeile 2 - Seite 17, Zeile 14; Seite 19, Zeile 12 - Seite 20, Zeile 18; Seite 24, Zeile 15 - Seite 25, Zeile 10; Seite 36, Zeile 16 - Seite 38, Zeile 14; Seite 39, Zeile 20 - Seite 40, Zeile 7; Seite 42, Zeile 18 - Seite 44, Zeile 3; Abbildungen 1-3,15-15 * | 1,2,4,10 | |
| A | --- | 3,5,7 | |
| Y | EP-A-0 340 145 (T.J. FOGARTY) * Seite 1, Zeilen 43-48; Seite 3, Zeile 43 - Seite 4, Zeile 37; Abbildungen 1,3 * | 1,2,4,10 | |
| A | --- | 6,9,11 | RECHERCHIERTE SACHGEBIETE (Int. Cl.5) |
| A | US-A-4 844 052 (K. IWAKOSHI et al.) * Spalte 1, Zeile 61 - Spalte 2, Zeile 10; Spalte 2, Zeile 39 - Spalte 3, Zeile 48; Abbildungen 1,4 * --- | 1,2,4,5,8,10 | A 61 B |
| A | DE-A-3 507 135 (OLYMPUS OPTICAL CO., LTD) * Seite 9, Zeile 15 - Seite 11, Zeile 5; Seite 12, Zeilen 6-11; Seite 13, Zeilen 6-13; Seite 16, Zeilen 1-28; Abbildungen 1-5 * --- -/- | 1-5,7,11 | |

Der vorliegende Recherchenbericht wurde für alle Patentansprüche erstellt

| Recherchenort DEN HAAG | Abschlußdatum der Recherche 18-04-1991 | Prüfer FONTENAY P.H.E.V. |

**Europäisches Patentamt**

# EUROPÄISCHER RECHERCHENBERICHT

Nummer der Anmeldung

EP 91 10 0157

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | DE-A-3 909 290 (ASAHI KOGAKU KOGYO K.K.)<br>* Spalte 3, Zeile 19 - Spalte 5, Zeile 21; Abbildungen 1-4 *<br>--- | 1-4,11 | |
| A | EP-A-0 075 153 (OLYMPUS OPTICAL CO., LTD)<br>* Seite 2, Zeilen 31-36; Seite 4, Zeile 34 - Seite 5, Zeile 33; Seite 7, Zeilen 1-31; Abbildungen 1-10 *<br>----- | 1,2,4,5,10 | |
| | | | RECHERCHIERTE SACHGEBIETE (Int. Cl.5) |

Der vorliegende Recherchenbericht wurde für alle Patentansprüche erstellt

| Recherchenort | Abschlußdatum der Recherche | Prüfer |
|---|---|---|
| DEN HAAG | 18-04-1991 | FONTENAY P.H.E.V. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

                                                    

& : Mitglied der gleichen Patentfamilie, übereinstimmendes Dokument

EPO FORM 1503 03.82 (P0403)